Europäisches Patentamt

European Patent Office

Office européen des brevets

(11) Publication number: **0 107 212**
**B1**

(12) EUROPEAN PATENT SPECIFICATION

(45) Date of publication of patent specification: 09.07.86

(51) Int. Cl.⁴: **C 12 N 11/08,** C 12 N 11/06

(21) Application number: 83111153.9

(22) Date of filing: 02.03.81

(60) Publication number of the earlier application in accordance with Art. 76 EPC: **0 059 182**

(54) **A process for the immobilization of an aminoacylase enzyme.**

(30) Priority: 17.07.80 HU 178280

(43) Date of publication of application:
02.05.84 Bulletin 84/18

(45) Publication of the grant of the patent:
09.07.86 Bulletin 86/28

(84) Designated Contracting States:
AT CH DE FR GB LI LU NL SE

(56) References cited:
FR-A-2 198 954
GB-A-1 257 263
GB-A-1 289 549
US-A-3 794 563
US-A-3 933 589

CHEMICAL ABSTRACTS, vol. 93, 1980, page
295, no. 200119c, Columbus, Ohio, USA B.
SZAJANI et al.: "Comparative studies on
soluble and immobilized pig kidney
aminoacylase"

(73) Proprietor: REANAL FINOMVEGYSZERGYAR
P.O. Box 54 Telepes u. 53
H-1441 Budapest (HU)

(72) Inventor: Boros, László, Dr.
Pauler u. 4
H-1013 Budapest (HU)
Inventor: Daróczi, Iván
Dessewffy u. 28
H-1066 Budapest (HU)
Inventor: Huber, Irén
Villányi u. 86
H-1118 Budapest (HU)
Inventor: Ivony, Józsefné
Mezö Imre u. 21-23
H-1081 Budapest (HU)
Inventor: Kiss, Jánosné
Vajda u. 36b
H-6723 Szeged (HU)
Inventor: Szajáni, Béla, Dr.
Tusnádi u. 26
H-1125 Budapest (HU)

(74) Representative: Beszédes, Stephan G. Dr.
Münchener Strasse 80a Postfach 1168
D-8060 Dachau (DE)

(58) References cited:
**BIOLOGICAL ABSTRACTS, vol. 73, 1982, no. 53185 B. SZAJANI et al.: "Preparation and practical utilization of a highly active immobilized form of porcine kidney aminoacylase"**

**Description**

The invention relates to a process for the immobilization of an aminoacylase enzyme.

Several methods have been disclosed so far to immobilize aminoacylase starting from enzymes isolated from pig kidney or of microbial origin. According to the methods discussed in J. Am. Chem. Soc. *81*, 4024 (1959) and Coll. Czech. Chem. Commun. *36*, 2398 (1971) aminoacylase is attached to DEAE cellulose by ionic bonds. Aminoacylase can also be immobilized, however, by covalent bonds. According to one of the known methods an acrylamide/methacrylate copolymer cross-linked with N,N - methylene - bis - (acrylamide) is applied as a support; the support is treated with hydrazine, then activated with sodium nitrite in the presence of hydrochloric acid, and finally the enzyme is coupled to the resulting acid azide (Macromolecules *4*, 350 [1971]). According to another known method an Enzacryl AA® type support (a polyacrylamide derivative containing aromatic amino groups) is activated with sodium nitrite in the presence of hydrochloric acid, and the enzyme is coupled to the resulting diazo derivative (Coll. Czech. Chem. Commun. *38*, 943 [1973).

These known methods of immobilization have the common disadvantage that they provide immobilized enzyme preparations with low specific activity.

Furthermore from French Application Print 2,198,954 it has been known to immobilize enzymes including the enzyme aminoacylase by combining the enzyme, a non-protein bonding agent, a cross-linking agent and water and/or organic solvents in whatever sequence as well as washing the obtained product with water. There may be used as bonding agents polyethyleneimines or polyamines and as cross-linking agents carbodiimides the preferred sequence is to combine first the enzyme in the water and/or organic solvent with the bonding agent and only this combination thereafter with the cross-linking agent.

Moreover from US—Patent 3,794,563 it has been known the use of polymers of acrylamide cross-linked among others with N - methylene - bis - acrylamide for the immobilization of enzymes. In column 1, line 69 to column 2, line 2 of this publication it has been stated that the covalent bonding of the enzyme to the polymer has the inconvenience that the reactive conditions required for the covalent bonding have to be carefully controlled in order to avoid destruction of the enzyme molecule and it is usually difficult to strip exhausted enzyme from the polymer to enable the polymer to be re-charged with fresh enzyme.

Furthermore from US—Patent 3,933,589 it has been known a process for immobilizing an enzyme on a support, wherein an enzyme, a support and a chemical immobilizing agent are maintained in contact at a temperature and for a time sufficient to chemically immobilize said enzyme, as a chemical immobilizing agent a preformed reaction solution of an alkane diamine and alkane dihalide being used.

Moreover from British Patent 1 289 549 it has been known a process for the preparation of a water insoluble enzyme preparation which process comprises reacting the enzyme dissolved or suspended in an aqueous solution with a cellulose carbonate or a diazotized diaminobenzene derivative thereof as a disadvantage of this process of immobilization on page 1, lines 50 to 53 of British Patent 1 257 263 there have been indicated the low yields of fixed proteins, the not secure attachment of the proteins and their denaturation during attachment.

Furthermore in British Patent 1 257 263 it has been described a process for producing articles containing active protein substances, in which a substrate in the definition of which aminoacylase has not been mentioned is contacted with a solution of at least one active protein substance in the presence of a bridging agent, at least the active protein substance(s) being adapted to react with the bridging agent, whereby covalently bonded bridges are formed between molecules of the active protein substance(s), with or without the formation of such bridges between the substrate and the active protein substance(s), and the substrate together with the active protein agents substance(s) is then isolated. Although in British Patent 1 257 263 the use of carbodiimides as bridging agents has been mentioned the bridging agent always is reacted with the enzyme without before having been bound to the substrate as it appears, for example, from page 7, lines 10 to 21 (in claim 1) of British Patent 1 257 263.

Moreover in Journal of Applied Biochemistry 1980, 2(1), pages 72 to 80 it has been described the immobilization of aminoacylase by an activated polyacrylamide-type support medium containing carboxylic functional groups. However, no mention has been made of the preparation of the support by partial hydrolysis of the amide groups of polyacrylamide nor of the activation of the support by carbodiimides. In this regard it is to be noted that polyacrylamide derivatives containing carboxylic groups also can be prepared by copolymerization of acryl amide/ N,N' - methylene - bis - (acrylamide) and acrylic acid. There exists also several activating methods different from the activation by carbodiimides, for example in the form of acid azides via acid hydrazides.

The problem underlying to the invention is by eliminating the said disadvantages of the known methods to provide for a process for the immobilization of an aminoacylase enzyme resulting in an immobilized enzyme preparation having a much greater specific activity than that of the known ones.

Now it has been found, unexpectedly, that an immobilized enzyme preparation with a specific activity greater by at least two orders of magnitude than that of the known ones can be

obtained, when aminoacylase enzyme is immobilized on a support prepared by the partial hydrolysis of an Akrilex® P type copolymer of acryl amide and N,N' - methylene - bis - (acrylamide).

The Akrilex® P type acryl amide/N,N' - methylene - bis - (acrylamide) copolymer beads (Akrilex® P-30, P-100 and P-200) are marketed by the Hungarian firm Reanal Finomvegyszergyár. When this copolymer is hydrolyzed with an acid (such as hydrochloric acid or another strong acid) or an alkali (such as sodiumhydroxide, sodium carbonate or another strong base), about 50% of the —CO—NH$_2$ groups are converted to carboxy groups, whereas the remaining —CO—NH$_2$ groups do not hydrolyze even under severe reaction conditions. Consequently, unchanged —CO—NH$_2$ groups are situated between carboxy groups in the hydrolyzed copolymer which fix the carboxy groups in favourable steric positions. If aminoacylase enzyme is coupled to the carboxy groups by a carbodiimide activation method known per se, the individual immobilized enzyme molecules do not interfere with the function of each other due to the favourable steric positions of the carboxy groups; thus the immobilized enzyme preparation possesses a very high specific activity.

Based on the above, the subject-matter of the invention is a process for the immobilization of an aminoacylase enzyme by activating a partially hydrolyzed copolymer, prepared by subjecting an Akrilex® P type acrylamide/N,N' - methylene - bis - (acrylamide) copolymer to, optionally alkaline, hydrolysis and applying to it the aminoacylase, which is characterized in that a partially hydrolyzed Akrilex® P type acrylamide/N,N' - methylene - bis - (acrylamide) copolymer which may be prepared beyond by alkaline hydrolysis alternatively by acidic hydrolysis is treated in a manner known per se with a carbodiimide compound which is soluble in water or soluble in an organic solvent at a temperature below 0°C, a solution of the aminoacylase with a pH of 6.5 to 8.5 is applied onto the resulting activated support and in a manner known per se the resulting product is washed and then, if desired dried.

Beyond the fact that in the process according to the invention a partially hydrolyzed Akrylex® P type acrylamide/N,N' - methylene - bis - (acryl-amide) copolymer is used as a support, whereas according to French Application Specification 2,198,954 such cannot be used but only poly-ethyleneimines or polyamines there is the fundamental difference between the process according the invention and the process according to French Application Specification 2,198,954 that in the former the carboxy groups of the acryl-amide/acrylic acid/N,N' - methylene - bis - (acrylamide) copolymer prepared by partial hydrolysis of the support acrylamide/N,N' - methylene - bis - (acrylamide) copolymer are activated by the carbodiimide compound and the so obtained derivative of the type of O-acyli-sourea reacts with the primary amino groups of the enzyme, whereas in the process of French Application Specification 2,198,954 by mixing the polymers containing imino or amino groups, the carbodiimide compound and the enzyme under corresponding conditions the carboxy groups of the asparagyl and/or glutamyl side chains of the enzyme furnish with the carbodiimide compound a derivative of the type O-acylisourea which reacts with the imino or amino groups of the support in the latter case there being the disadvantage that it cannot be excluded the formation of bonds between activated enzyme molecules or within the enzyme molecules, respectively, which impairs the degree of efficiency of the immobilization reaction, whereas the reaction in the process according to the invention is strictly controlled.

The process according to the invention is still more remote from the process of US—Patent 3,794,563 since in the latter no cross-linking with carbodiimide compounds at all is carried out and moreover the aminoacylase has not been mentioned as an enzyme.

Still more remote from the process according to the invention is US—Patent 3,933,589 since the supports used according to the invention are entirely different from those used in the US—Patent 3,933,589 which also applies to the carbodiimide compound used in the process according to the invention as opposed to the reaction solution of an alkane dihalide and an alkane diamine used according to the said publication which has nothing to do with the carbodiimide compound used in the process according to the invention.

There exists the fundamental difference between the immobilization process according to the invention and that according to British Patent 1 289 549 that in the first case as opposed to the last case in which cellulose carbonate or a diazotized diaminobenzene derivative of it has been employed a partially hydrolyzed acryl amide/N,N' - methylene - bis - (acrylamide) copolymer is used. Thereby the disadvantages of the products of British Patent 1 289 549 already mentioned, namely low yields of fixed proteins, not secure attachment of the proteins and denaturation of them during attachment, do not occur in the process according to the invention. Thus by the process of immobilization according to the invention superior yields (76%) of immobilized aminoacylase enzyme are attained and the resulting covalent bond is strong (can be split only by boiling in a strongly acid or alkaline medium).

Contrary to the process according to British Patent 1 257 263 in which the bridging agent always is reacted with the enzyme without before having been bound to the substrate in the process of immobilization according to the invention first the carbodiimide compound is bound to the acryl amide/N,N' - methylene - bis - (acrylamide) copolymer and only the so obtained material is treated with a solution of the aminoacylase. Thus from British Patent 1 257 263 not even the greatest part of the entering clause of the process of immobilization according to the invention has been known, not to mention the characterizing part of it. On account of these differences the process according to the invention also as compared to British Patent 1 257 263 involves the differences already discussed above as compared to other processes of immobilization.

The partially hydrolyzed acryl amide/N,N' - methylene - bis - (acrylamide) copolymer used in the process of immobilization according to the invention has much more advantageous properties than other supports, such as those obtained by copolymerization of acryl amide/ N,N' - methylene - bis - (acrylamide) and acrylic acid. Moreover the activity of aminoacylase immobilized by the process according to the invention is by 2 orders of magnitude higher than that of aminoacylase activated, for example, in form of acid azide via acid hydrazide. Hence all the more because of the other activating possibilities by which poor results have been obtained the process of immobilization of aminoacylase according to the invention by binding the aminoacylase to the O-acylisourea intermediate which is prepared by the activation of the carboxyl groups of the support prepared by partial hydrolysis of the polyacrylamide acryl amide/ N,N' - methylene - bis - (acrylamide) copolymer by means of a carbodiimide compound is based on an inventive activity also as compared to J. Appl. Biochem. 1980, 2(1), pages 72 to 80.

Aminoacylase of any origin, isolated by any method can be applied as enzyme in the above process according to the invention it is preferred, however, to utilize as a starting substance an enzyme separated from the kidneys of mammals according to the new process of European Patent Application 81 900 726.1 (publication number 59 182).

To activate the partially hydrolyzed Akrilex® P type copolymer, e.g. 1 - cyclohexyl - 3 - (2' - morpholinoethyl) - carbodiimide metho - p - toluenesulfonate or 1 - ethyl - 3 - (3' - dimethylaminopropyl) - carbodiimide, can be applied as a carbodiimide compound. It is preferred to utilize water-soluble carbodiimides for this purpose. From the carbodiimide compounds soluble only in organic solvents those substances can be applied in the process of the invention which are appropriately soluble in the given organic solvent at below 0°C.

Aminoacylase is applied onto the activated support from a solution of pH 6.5 to 8.5, preferably from an almost neutral solution (pH about 7.0). Aminoacylase is applied preferably as a solution formed with a 0.1 molar potassium phosphate buffer (pH=7.0).

The immobilized enzyme preparation obtained in the coupling reaction is washed in a manner known per se, and then dried, if desired. The enzyme preparation can also be stored, however, as an aqueous suspension in the presence of an appropriate stabilizing agent, such as sodium azide.

The specific activity of the immobilized enzyme preparations prepared according to the invention is 150 to 200 units/mg of xerogel, which, when compared to the specific activity of 0.55 to 2.24 units/mg of xerogel attainable by the method discussed in Macromolecules 4, 350 (1971), represents an increase in activity of at least two orders of magnitude.

When examining the new immobilized enzyme preparations prepared according to the invention it has been found, unexpectedly, that the stability of the enzyme in the alkaline pH region increases upon immobilization.

This phenomenon is very advantageous when the immobilized enzyme preparation is applied for the enzymatic resolution of DL-amino acids. It is known that the enzymatic resolution of DL-amino acids proceeds with the highest rate at about pH 7.5, and the reaction rate decreases with decreasing pH value. Since the pH of the reaction mixture always shifts to the acidic region with the progress of the resolution, the reaction rate continuously decreases with time. If the new enzyme preparations prepared according to the invention with increased stability in the alkaline pH region are applied to resolve DL-amino acids, resolution can be started at a pH higher than the usual, thereby compensating for the decelerating effect of the continuous decrease in pH.

The invention is elucidated in detail by the aid of the following non-limiting Example.

Example

30 g of an Akrilex® P-100 xerogel are suspended in 1 litre of a 1 n aqueous sodium hydroxide solution, and the suspension is maintained at 50°C for 3 hours. Thereafter the swollen gel is washed with 12 litres of distilled water until neutral, and then washing is continued with 10.5 litres of a 0.05 n aqueous hydrochloric acid. Thereafter the gel is washed with 30 litres of distilled water, and then freeze-dried. 26 g of a support material are obtained; binding capacity: 6.14 milliequivalents/g. This support material is further treated as follows:

Alternative a)

10 g of the xerogel prepared as described above are suspended in 500 ml of a potassium phosphate buffer (pH=7.0), and a solution of 20 g of 1 - cyclohexyl - 3 - (2' - morpholinoethyl) - carbodiimide metho - p - toluenesulfonate in 250 ml of a cold (+4°C) buffer solution is added to the suspension under steady stirring and cooling with an ice water bath. After 10 minutes of stirring a solution of 5 g of an aminoacylase, prepared as described in Example 3, of parent European Patent 59182, in 250 ml of a cold (+4°C) buffer solution is added, and the resulting suspension is stirred at +4°C for 48 hours. The gel is separated and washed in sequence thrice with 1 litre portions of a 0.1 molar potassium phosphate buffer (pH=7.0), thrice with 1 litre portions of a 0.1 molar potassium phosphate buffer also containing 0.5 moles of sodium chloride (pH=7.0), and then thrice with 1 litre portions of a 0.1 molar potassium phosphate buffer. The gel is washed salt-free four times with 2.5 litre portions of distilled water, and then subjected to freeze-drying. 18 g of an immobilized aminoacylase preparation are obtained. Activity: hydrolysis of 158 μmoles of N - acetyl - L - methionine/hour/mg of dry substance.

Alternative b)

10 g of the xerogel prepared as described above are suspended in 500 ml of a 0.1 molar potassium phosphate buffer (pH=7.0), and a solution of 10 g of 1 - ethyl - 3 - (3' - dimethylaminopropyl) - carbodiimide in 250 ml of a cold (+4°C) buffer solution is added to the suspension under steady stirring and cooling with an ice water bath. After 10 minutes of stirring a solution of 5 g of an aminoacylase, prepared as described in Example 3 of parent European Patent 59182, in 250 ml of a cold (+4°C) buffer solution is added, and the resulting suspension is stirred at +4°C for 48 hours. The gel is separated, and washed in sequence thrice with 1 litre portions of a 0.1 molar potassium phosphate buffer, thrice with 1 litre portions of a 0.1 molar potassium phosphate buffer also containing 0.3 moles of sodium chloride, and then thrice with 1 litre portions of a 0.1 molar potassium phosphate buffer (pH=7.0). The gel is washed salt-free four times with 2.5 litre portions of distilled water, and then subjected to freeze-drying. 16 g of an immobilized aminoacylase preparation are obtained. Activity: hydrolysis of 202 μmoles of N - acetyl - L - methionine/hour/mg of dry substance.

## Claim

A process for the immobilization of an aminoacylase enzyme by activating a partially hydrolyzed copolymer, prepared by subjecting an acryl amide/N,N' - methylene - bis - (acryl-amide) copolymer to, optionally alkaline, hydrolysis and applying to it the aminoacylase, characterized in that a partially hydrolyzed acryl-amide/N,N' - methylene - bis - (acrylamide) copolymer, in which copolymer when hydrolyzed with an acid or an alkali about 50% of the —CO—NH$_2$ groups are converted to carboxy groups, whereas the remaining —CO—NH$_2$ groups do not hydrolyze even under severe reaction conditions (Akrilex® P type) and the hydrolyzed copolymer may be prepared beyond by alkaline hydrolysis alternatively by acidic hydrolysis, is treated in a manner known per se with a carbodiimide compound which is soluble in water or soluble in an organic solvent at a temperature below 0°C, a solution of the aminoacylase with a pH of 6.5 to 8.5 is applied onto the resulting activated support and in a manner known per se the resulting product is washed and then, if desired, dried.

## Patentanspruch

Verfahren zur Immobilisierung eines Aminoacylaseenzyms durch Aktivieren eines teilweise hydrolysierten Copolymeren, hergestellt durch Unterwerfen eines Acrylamid/N,N' - Methylen - bis - (acrylamid) - Copolymeren einer gegebenenfalls alkalischen Hydrolyse und Aufbringen einer Aminoacylase auf dasselbe, dadurch gekennzeichnet, daß ein teilweise hydrolysiertes Acrylamid/N,N' - methylen - bis - (acrylamid) - Copolymeres, wobei in dem Copolymeren, falls es mit einer Säure oder einem Alkali hydrolysiert wird, ungefähr 50% der —CO—NH$_2$-Gruppen in Carboxygruppen umge-wandelt werden, während die restlichen —CO—NH$_2$-Gruppen auch nicht unter scharfen Reaktionsbedingungen hydrolysieren (Akrilex® P-Typ), und das hydrolysierte Copolymere außer durch alkalische Hydrolyse wahlweise durch saure Hydrolyse heregestellt werden kann, in an sich bekannter Weise mit einer Carbodiimidverbindung behandelt wird, die in Wasser oder in einem organischen Lösungsmittel bei einer Temperatur unter 0°C löslich ist, eine Lösung der Aminoacylase mit einem pH von 6,5 bis 8,5 auf den erhaltenen Träger aufgebracht wird und in an sich bekannter Weise das erhaltene Produkt gewaschen und dann gegebenenfalls getrocknet wird.

## Revendication

Procédé pour l'immobilisation d'une aminoacylase par activation d'un copolymère partiellement hydrolysé, préparé en soumettant un copolymère d'acrylamide/N,N' - méthylène - bis - (acrylamide) à une hydrolyse éventuellement alcaline et application de l'aminoacylase à celui-ci, caractérisé en ce que l'on traite selon une manière connue en elle-même, un copolymère d'acrylamide - /N,N' - méthylène - bis (acryl-amide) partiellement hydrolysé en lequel copolymère en cas qu'est hydrolysé avec un acide ou un alcali environ 50% des groupes —CO—NH$_2$— sont transformés en groupes carboxy tandis que les groupes —CO—NH$_2$— restants ne sont pas hydrolysés même dans conditions de réaction sévères (type Akrilex® P) et le copolymère hydrolysé qui peut être préparé ainsi par hydrolyse alcaline dans une variante par hydrolyse acide, avec un composé carbodiimide qui est soluble dans l'eau ou soluble dans un solvant organique à une témpérature inférieure à 0°C, qu'on applique une solution de l'aminoacylase ayant un pH de 6,5 à 8,5 sur le support activé résultant, puis que l'on lave le produit résultant d'une manière connue et qu'on le sèche ensuite si cela est désiré.